# EUROPÄISCHE PATENTSCHRIFT

(11) **EP 0 942 282 B1**
(45) Veröffentlichungstag und Bekanntmachung des Hinweises auf die Patenterteilung: **13.08.2003**
(21) Anmeldenummer: 99103874.6
(22) Anmeldetag: 01.03.1999
(51) Int. Cl.: G01N 33/36

(54) **Vorrichtung zum Messen von Eigenschaften eines textilen Produktes**
Apparatus for measuring characteristics of a textile product
Appareil pour mesurer des caractéristiques d'un produit textile

(30) Priorität: 13.03.1998 CH 61398
(43) Veröffentlichungstag der Anmeldung: 15.09.1999
(73) Patentinhaber: Uster Technologies AG, 8610 Uster (CH)
(72) Erfinder: Zehr, Jürg, 8610 Uster (CH); Madone, Diego, 8634 Hombrechtikon (CH); Isotton, Walter, 8610 Uster (CH)
(74) Vertreter: Ellenberger, Maurice

(56) Entgegenhaltungen:
- EP-A- 0 266 614
- EP-A- 0 578 975
- DE-A- 2 437 738
- DE-A- 2 831 242
- DE-A- 4 025 899
- US-A- 3 788 138

## Beschreibung

Die Erfindung betrifft eine Vorrichtung zum Messen von Eigenschaften eines textilen Prüfgutes in einem Messspalt, in dem das textile Prüfgut eingeführt ist, wobei der Messspalt durch zwei Wände gebildet ist und zur Führung des Prüfguts im Messspalt je ein Führungselement vorgesehen ist.

Eine solche Vorrichtung ist beispielsweise aus der EP 0 266 614 bekannt, bei der in einem Messkamm mehrere, verschieden breite Messspalte angeordnet sind, durch die das Prüfgut geführt wird. Die Führung des Prüfgutes in den Messspalten wird dabei durch eine Fadenführungseinrichtung und durch eine Vorschubeinrichtung besorgt, die beide in separaten und auswechselbaren Modulen angeordnet sind, die vom Modul mit dem Messkamm trennbar sind. Die Fadenführungseinrichtung und die Vorschubeinrichtung spannen zusammen das Prüfgut, so dass es zwischen diesen Elementen und damit auch im Messspalt einen geradlinigen Verlauf nimmt. Sowohl die Fadenführungseinrichtung wie auch die Vorschubeinrichtung sind in einem gewissen Abstand zum Messspalt angeordnet.

Eine weitere Vorrichtung ist aus der US 3,788,138 bekannt, bei der je ein homähnliches Führungselement oberhalb und unterhalb eines Messkammes angeordnet ist. Diese beiden Führungselemente können quer zu den Messspalten des Messkammes verschoben und in beliebigen Stellungen dazu befestigt werden. Dabei müssen die Führungselemente so eingestellt werden, dass sie einerseits das Prüfgut seitlich auslenken, so dass es gut auf den Führungselementen anliegt und dass sie andererseits das Prüfgut im Messspalt so führen, dass es schön parallel zu den Wänden des Messspaltes und möglichst in dessen Mitte durchläuft.

Ein Nachteil der genannten Vorrichtungen ist darin zu sehen, dass die Führungseinrichtung genau so eingestellt werden muss, dass sich das Prüfgut möglichst genau an einem vorbestimmten Ort, z.B. in der Mitte des Messspaltes befindet, wenn es durch diesen hindurchbewegt wird. Dieses Einstellen der Führungseinrichtung muss mit grosser Sorgfalt und Genauigkeit vorgenommen werden, da beispielsweise bei Vorrichtungen, die zum Prüfen von Garn bestimmt sind, die Breite eines Messspaltes 1mm oder weniger beträgt. Zudem ist es schwierig von Auge die Verhältnisse im Messspalt zu überblicken und die vorhandenen Abstände zu erkennen. Die Lage der Führung oder des Messspaltes muss jedesmal neu eingestellt werden, nachdem das Modul mit der Führung oder dem Messkamm aus- und eingebaut wurde und wenn ein neues Prüfgut mit einem anderen Durchmesser geprüft werden soll.

Die Erfindung, wie sie in den Ansprüchen gekennzeichnet ist, löst die Aufgabe, eine Vorrichtung der genannten Art zu schaffen, bei der das Einstellen der Führung erleichtert ist und bei der das Prüfgut trotzdem genau genug geführt ist.

Dies wird gemäss der Erfindung, die durch die Merkmale das Anspruchs 1 definiert ist, erreicht.

Die durch die Erfindung erreichten Vorteile sind insbesondere darin zu sehen, dass keine Massnahmen getroffen werden müssen, um das Führungselement und den Messspalt nachträglich gegeneinander auszurichten. Die Einstellung erfolgt einmal für sehr lange Zeit, z.B. bis das Führungselement eine Abnützung durch das Prüfgut aufweist, die im Vergleich zur Breite des Messspaltes gewichtig ist. Die erfindungsgemässe Art der Führung eignet sich sowohl für einzelne Messspalte wie auch für mehrere nebeneinanderliegende Messspalte, die zusammen einen sogenannten Messkamm bilden. Die Führungen können so ausgebildet sein, dass sie der Bewegung des Prüfgutes möglichst wenig Widerstand entgegensetzen.

Im folgenden wird die Erfindung anhand von Beispielen und mit Bezug auf die beiliegenden Figuren näher erläutert. Es zeigen:
Figur 1 eine erfindungsgemässe Vorrichtung mit mehreren Messspalten,
Figuren 2 bis 4 sowie 9 und 10 je eine vereinfachte Ansicht eines Messspaltes,
Figuren 5 und 6 sowie 11 und 12 je eine vereinfachte Ansicht mehrerer nebeneinanderliegender Messspalte,
Figuren 7 und 8 je einen Teil eines Messspaltes und
Figur 13 einen Teil des Messspaltes.

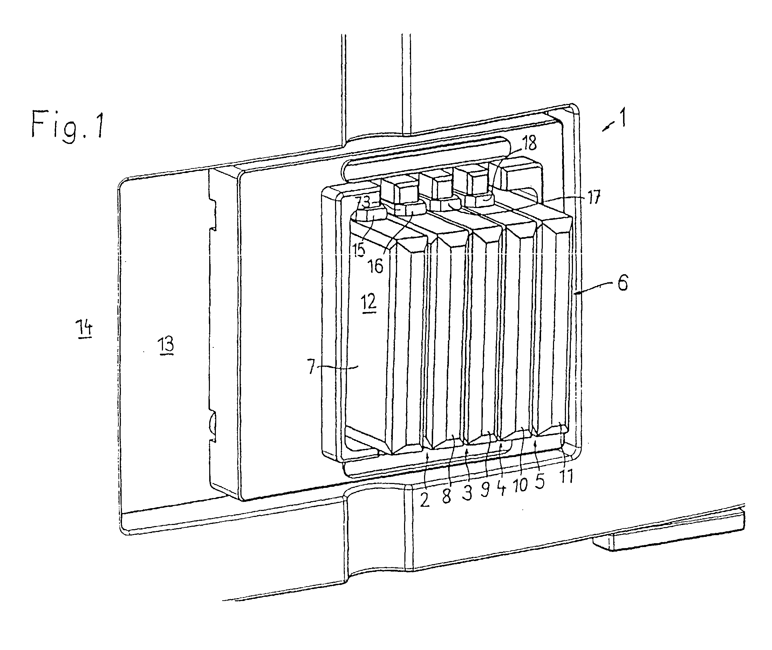

Fig. 1 zeigt einen Teil 1 einer Vorrichtung zum Messen von Eigenschaften eines textilen Prüfgutes mit mehreren Messspalten 2, 3, 4 und 5, die zusammen einen Messskamm 6 bilden. Die Messspalte 2 bis 5 liegen zwischen plattenförmigen Elementen 7, 8, 9, 10 und 11, die beispielsweise als Träger für hier nicht sichtbare Elektroden von Kondensatoren oder Messzellen dienen. Die Messspalte 2 bis 5 sind beidseitig jeweils durch Wände begrenzt, die hier kaum sichtbar und deshalb nicht näher bezeichnet sind. Diese Wände entsprechen aber der hier sichtbaren Wand 12 des Elementes 7 und tragen zusätzlich beispielsweise Elektroden als Messelemente wie dies beispielsweise in der schweizerischen Patentanmeldung Nr. 2926/97 beschrieben ist. Der Messkamm 6 ist hier auf einer Trägerplatte 13 befestigt, die wiederum in einem Gehäuse 14 angeordnet ist. Der hier gezeigte Teil 1 ist beispielsweise Teil eines Garnprüfgerätes, wie es zur Prüfung von Eigenschaften von Gamen bekannt ist.

Jedem Element 7, 8, 9, 10 und damit auch den Wänden dieser Elemente, ist eines der hier gezeigten Führungselemente 15, 16, 17 und 18 zugeordnet. Diese Führungselemente 15 bis 18 sind je auf einem der Elemente 7 bis 10 befestigt oder mindestens ortsfest dazu angeordnet, so dass diese auch eine feste Lage zu einer Wand einnehmen. Zwischen den Wänden oder Elementen 7 bis 11 soll ein Prüfgut, beispielsweise ein Garn, ein Band, ein Filament usw. in seiner Längsrichtung in an sich bekannter und deshalb hier nicht näher dargestellter Art und Weise, in Fig. 1 beispielsweise von oben nach unten, bewegt werden. Dabei bewegt sich das Prüfgut auf einem der Führungselemente 15 bis 18. Wenn auch hier nicht ersichtlich, so sind doch auch am unteren Ende der Messspalte 2 bis 5 ebenfalls Führungselemente angeordnet. Die Führungselemente 15 bis 18 können beispielsweise so ausgeformt sein, dass sie das Einlegen des Prüfgutes erleichtern. Dies geschieht beispielsweise mit Anschrägungen, wie hier beispielsweise am Führungselement 16 mit 73 bezeichnet. Die Elemente 7 bis 11 und die Führungselemente 15 bis 18 können sehr verschieden ausgebildet und zueinander angeordnet sein, wie dies aus den nachfolgend beschriebenen Figuren hervorgehen soll.

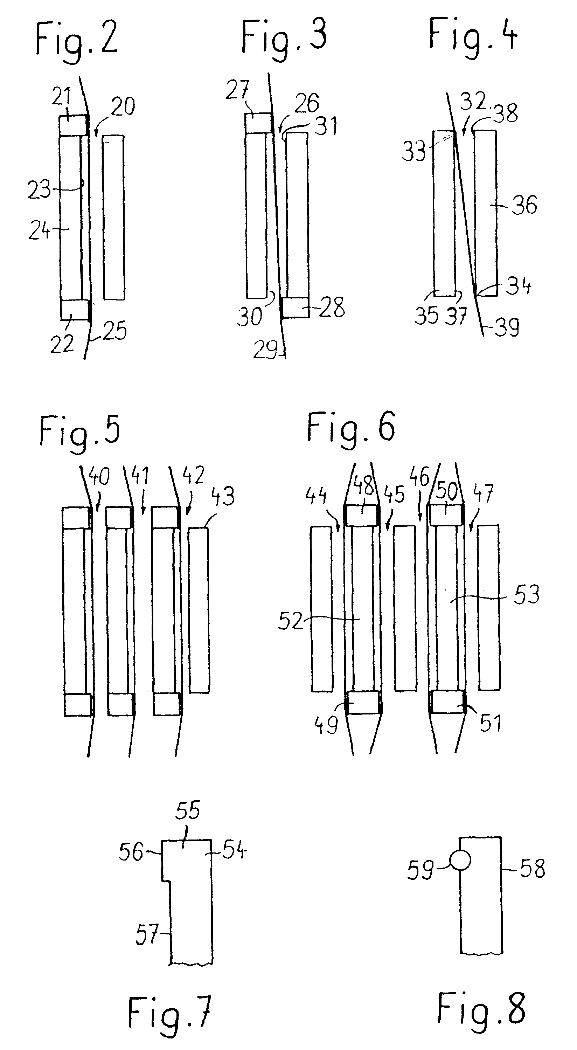

Fig. 2, die Kein Teil der vorliegenden Erfindung ist, zeigt einen Messspalt 20 mit Führungselementen 21, 22, die beide derselben Wand 23 oder demselben plattenförmigen Element 24 zugeordnet sind und insbesondere auf diesen und von der Wand 23 vorstehend auf dieser angeordnet sind. Dadurch wird das Prüfgut 25 in einem definierten Abstand zur einen Wand 23 geführt. Hier stehen beide Führungselemente 21, 22 gleichviel vor, so dass das Prüfgut 25 parallel zur Wand 23 liegt.

Fig. 3 zeigt einen Messspalt 26 mit Führungselementen 27, 28, die, bezogen auf den Messspalt 26, in Längsrichtung und in Querrichtung gesehen auf der entgegengesetzten Seite des Messspaltes 26 liegen. In der gezeigten Anordnung, stehen die beiden Führungselemente 27 und 28 nicht bis zur Mitte des Messspaltes 26 vor, so dass das Prüfgut 29 zu den Wänden 30 und 31 nicht notwendigerweise parallel verläuft und eine andere für die Messung günstige Position einnimmt.

Fig. 4 zeigt einen weiteren Messspalt 32, bei dem Führungselemente 33, 34 in die Elemente 35, 36 integriert sind und so von den Wänden 37, 38 nicht hervorstehen. Das Prüfgut 39 liegt dabei nicht parallel zu den Wänden 37, 38 im Messspalt 32. Da beispielsweise die Elektroden einer Messzelle in einem Messspalt nicht die ganze Fläche der Wände 37, 38 beanspruchen und damit nur in gewisse Bereiche dieser Wände eingesetzt oder aufgebracht sind, kann das Trägermaterial, aus dem die Elemente 35, 36 bestehen, ganz oder nur lokal Eigenschaften erhalten, die zur Führung des Prüfguts 39 taugen.

Fig. 5, die Kein Teil der vorliegenden Erfindung ist, zeigt einen Messkamm mit mehreren Messspalten 40, 41, 42, die nicht dieselbe Breite aufweisen und in denen das Prüfgut, wie in Fig. 2 gezeigt, auf der gleichen Seite oder vor der gleichen Wand geführt ist. Deshalb weist ein Element 43 keine Führungselemente auf.

Fig. 6, die Kein Teil der vorliegenden Erfindung ist, zeigt einen Messkamm mit mehreren Messspalten 44, 45, 46 und 47, wobei Führungselemente 48, 49, 50 und 51 nur an zwei Elementen 52 und 53 angeordnet sind. Jedes Führungselement ist in diesem Falle doppelt wirkend ausgebildet, d.h. es hat auf zwei Seiten Führungsflächen für zwei Prüfgüter.

Fig. 7 zeigt einen Teil eines Elementes 54, bei dem das Führungselement 55 integriert ist und eine Führungsfläche 56 aufweist, die aus einer Wand 57 vorsteht.

Fig. 8 zeigt ein Führungselement 59, das in ein plattenförmiges Element 58 und dessen Wand eingesetzt und darin befestigt ist.

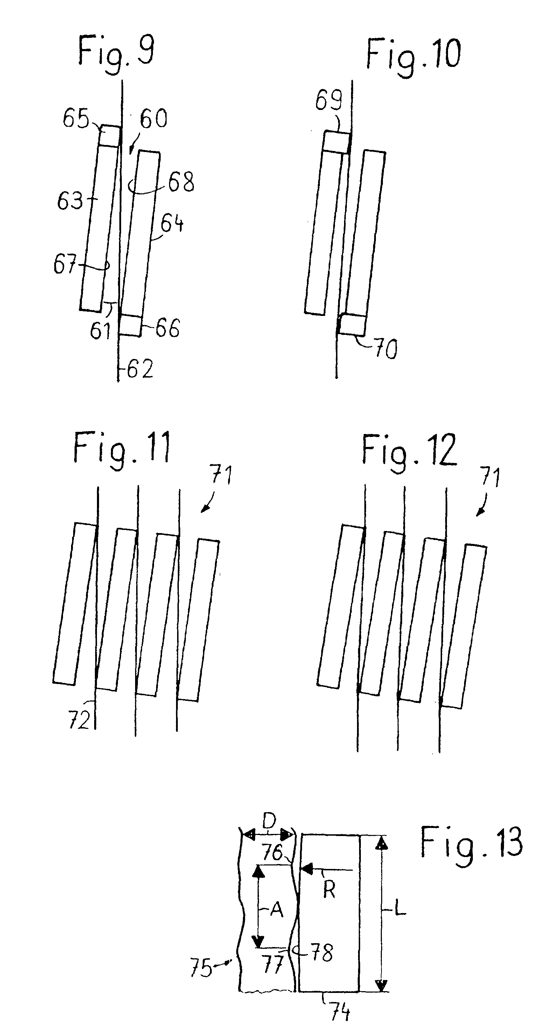

Fig. 9 zeigt einen Messspalt 60, der um einen Winkel 61 zum Prüfgut 62 geneigt ist. Den plattenförmigen Elementen 63 und 64 sind Führungselemente 65 und 66 zugeordnet, die aus Wänden 67 und 68 nicht hervorstehen.

Fig. 10 zeigt eine ähnliche Ausführung zu derjenigen gemäss Fig. 9 aber mit vorstehenden Führungselementen 69 und 70. Diese sind alternierend je an einem Ende eines plattenförmigen Elementes oder einer Wand angeordnet.

Fig. 11 zeigt einen Messkamm 71 mit integrierten Führungselementen gemäss Fig. 4 der so gerichtet ist, dass das Prüfgut 72 nicht ausgelenkt wird.

Fig. 12 zeigt denselben Messkamm 71 wie Fig. 11, der aber weiter geneigt ist, so dass das Prüfgut leicht ausgelenkt wird.

Selbstverständlich sind weitere Kombinationen insbesondere mit Gruppen von plattenförmigen Elementen und Führungselementen denkbar, die eine Ausführung im Sinne der Patentansprüche darstellen, wenn sie hier auch nicht ausdrücklich gezeigt sind.

Figur 13 zeigt eine spezielle Ausbildung eines Führungselementes 74 für ein Prüfgut 75, das beispielsweise periodische Unregelmässigkeiten aufweist, die sich hier beispielsweise als Erhebungen oder Einbuchtungen 76, 77 an dessen Oberfläche äussern. Ein Beispiel dafür ist ein aus mehreren Filamenten bestehendes, verdrehtes Garn. Um Schwingungen des in seiner Längsrichtung bewegten Prüfgutes zu vermeiden, soll die Länge L des Führungselementes 74 grösser sein als ein Abstand A zwischen den genannten Erhebungen oder Einbuchtungen. Der Radius R einer Führungsfläche 78 soll wesentlich grösser sein als der Durchmesser D des Prüfgutes. Besonders vorteilhaft ist ein Radius R der mindestens das Zwanzigfache dieses Durchmessers D beträgt.

Die Wirkungsweise der erfindungsgemässen Vorrichtung ist dabei wie folgt:

Für die Messung von Eigenschaften wird das Prüfgut in einen der Messspalte 2, 3, 4 oder 5 eingelegt und soweit nach hinten geschoben, bis er in den Bereich der Führungselemente 15 bis 18 gelangt. Durch den Antrieb wird das Prüfgut in seiner Längsrichtung bewegt und damit auch gespannt, so dass es sich über die Führungsflächen der Führungselemente an diesem Messspalt legt. Damit ist das Prüfgut im Messspalt genau positioniert und liegt an der richtigen Stelle im Messfeld des Messspaltes. Das Prüfgut kann, wie in den Figuren 3, 4 und 9 bis 12 gezeigt, sich quer durch die Breite des Messspaltes bewegen oder liegen. Diese Lage des Prüfgutes im Messspalt ist sehr vorteilhaft, weil sich das Prüfgut immer durch die Mitte des Messspaltes hindurch bewegt. Dies mindestens dann, wenn der Messspalt mit den Führungselementen symmetrisch aufgebaut ist. Damit besteht auch die Möglichkeit, den Messkamm schwenkbar auszubilden, um beispielsweise in einer Stellung, beispielsweise gemäss Fig. 1 oder 11, das Einführen des Prüfguts von vome zu erleichtern, und in einer anderen Stellung eine sichere Führung mit entsprechender Auslenkung des Prüfgutes zu gewährleisten.

## Patentansprüche

1. Vorrichtung zum Messen von Eigenschaften eines textilen Prüfgutes in einem Messspalt (2, 3, 4, 5), in dem das textile Prüfgut eingeführt ist, wobei der Messspalt durch zwei Wände gebildet ist und zur Führung des Prüfguts im Messspalt den beiden Wänden je ein Führungselement (15, 16, 17, 18) zugeordnet ist, **dadurch gekennzeichnet, dass** das einer Wand zugeordnete Führungselement mit dieser fest verbunden ist und dass die beiden Führungselemente am oberen und am unteren Ende des Messspaltes auf entgegengesetzten Seiten des Messspaltes so angeordnet sind, dass das Prüfgut nicht parallel zu den Wänden im Messspalt liegt.

2. Vorrichtung nach Anspruch 1, **dadurch gekennzeichnet, dass** das Führungselement (21, 22) auf die Wand (23) aufgesetzt ist.

3. Vorrichtung nach Anspruch 1, **dadurch gekennzeichnet, dass** das Führungselement (59) in die Wand eingesetzt ist.

4. Vorrichtung nach Anspruch 1, **dadurch gekennzeichnet, dass** das Führungselement (21) über die Wand (23) hinaus gegen das Prüfgut hin vorsteht.

5. Vorrichtung nach Anspruch 1, **dadurch gekennzeichnet, dass** das Führungselement (33, 34) einen Teil der Wand (37, 38) bildet und aus der Wand nicht hervorsteht.

6. Vorrichtung nach Anspruch 1, **dadurch gekennzeichnet, dass** der Messspalt (60) mit dem Führungselement (65, 66) zum Prüfgut (62) geneigt ist.

7. Vorrichtung nach Anspruch 1, **dadurch gekennzeichnet, dass** das Führungselement so ausgebildet und angeordnet ist, dass es das Prüfgut auslenkt.

8. Vorrichtung nach Anspruch 1, **dadurch gekennzeichnet, dass** mehrere Messspalte (2, 3, 4, 5) mit Führungsetementen (15, 16, 17, 18) nebeneinander angeordnet sind.

## Claims

1. Device for measuring properties of a textile test specimen in a measuring gap (2, 3, 4, 5), in which the textile test specimen is introduced, the measuring gap being formed by two walls and to both walls a guide element (2, 3, 4, 5) for the test specimen is associated, **characterised in that** the guide element is firmly connected to the wall to which it is associated and **in that** both guide elements are disposed at the top and at the bottom ends respectively on opposite sides of the measuring gap in such manner that the test specimen does not lie parallel to the walls of the measuring gap.

2. Device according to claim 1, **characterized in that** the guide element (21, 22) is placed onto the wall (23).

3. Device according to claim 1, **characterised in that** the guide element (59) is inserted into the wall.

4. Device according to claim 1, **characterised in that** the guide element (59) projects beyond the wall (23) in the directon of the test specimen.

5. Device according to claim 1, **characterised in that** the guide element (33, 34) forms a part of the wall (37, 38) and does not project beyond the wall.

6. Device according to claim 1, **characterised in that** the measuring gap (60) with the guide element (65, 66) is inclined relative to the test specimen (62).

7. Device according to claim 1, **characterised in that** the guide element is designed and disposed in such a way that it deflects the test specimen.

8. Device according to claim 1, **characterised in that** a plurality of measuring gaps (2, 3, 4, 5) each having a guide element (15, 16, 17, 18) are disposed adjacent to one another.

## Revendications

1. Dispositif pour mesurer des caractéristiques d'un échantillon textile de contrôle dans une fente de mesure (2, 3, 4, 5) dans laquelle l'échantillon textile de contrôle est inséré, où la fente de mesure est formée par deux parois et, pour le guidage de l'échantillon de contrôle dans la fente de mesure, il est associé aux deux parois respectivement un élément de guidage (15, 16, 17, 18), **caractérisé en ce que** l'élément de guidage associé à une paroi est relié solidement à celle-ci, et **en ce que** les deux éléments de guidage sont disposés à l'extrémité supérieure et à l'extrémité inférieure de la fente de mesure sur des côtés opposés de la fente de mesure de façon que l'échantillon de contrôle ne se situe pas parallèlement aux parois dans la fente de mesure.

2. Dispositif selon la revendication 1, **caractérisé en ce que** l'élément de guidage (21, 22) est placé sur la paroi (23).

3. Dispositif selon la revendication 1, **caractérisé en ce que** l'élément de guidage (59) est inséré dans la paroi.

4. Dispositif selon la revendication 1, **caractérisé en ce que** l'élément de guidage (21) fait saillie au-delà de la paroi (23) vers l'échantillon de contrôle.

5. Dispositif selon la revendication 1, **caractérisé en ce que** l'élément de guidage (33, 34) forme une partie de la paroi (37, 38) et ne dépasse pas de la paroi.

6. Dispositif selon la revendication 1, **caractérisé en ce que** la fente de mesure (60) avec l'élément de guidage (65, 66) est inclinée vers l'échantillon de contrôle (62).

7. Dispositif selon la revendication 1, **caractérisé en ce que** l'élément de guidage est réalisé et disposé de manière qu'il dévie l'échantillon de contrôle.

8. Dispositif selon la revendication 1, **caractérisé en ce que** plusieurs fentes de mesure (2, 3, 4, 5) avec des éléments de guidage (15, 16, 17, 18) sont disposées les unes à côté des autres.
